# EUROPEAN PATENT APPLICATION

(11) **EP 2 682 379 A1**
(43) Date of publication of application: **08.01.2014**
(21) Application number: 13150572.9
(22) Date of filing: 08.01.2013
(51) Int. Cl.: C07C 51/265, C07C 63/26

(54) **Method for producing terephthalic acid**

(30) Priority: 05.07.2012 CN 201210236322
(71) Applicant: Linde Aktiengesellschaft, 80331 München (DE)
(72) Inventor: Cha, Zhixiong, Scotch Plains, NJ New Jersey 07076 (US); Deng, Yunhui, 201206 Pudong Shanghai (CN); Xiu, Guohua, 201204 Pudong Shanghai (CN)
(74) Representative: Hofmann, Andreas

(57) **Abstract**

In order to overcome the problems that earlier methods have experienced, a method for producing terephthalic acid is proposed, comprising
- reacting paraxylene and oxygen in at least one air-based paraxylene oxidation stirred reactor in the presence of at least one catalyst, and
- adding, in particular injecting, at least one additional or auxiliary oxygen stream to the reactor.

## Description

### Technical field of the present invention

The present invention relates to a method for producing terephthalic acid.

### Background of the present invention; prior art

A number of methods have been practiced to improve the production of terephthalic acid, in particular of purified terephthalic acid (PTA). Amongst these methods are the oxidation of paraxylene (PX) by oxygen-enriched air and pure oxygen.

In a conventional Co-Mn-Br catalyzed air or oxygen-enriched air based paraxylene oxidation process for manufacturing terephthalic acid, the stirred reactor is operated from a temperature of about 170°C to about 225°C and a pressure of 100 psig [= hundred pounds per square inch gauge = 689.48 Kilopascal (kPa)] to 300 psig [= 2.07 Megapascal (MPa)].

Compressed air or oxygen-enriched air, enriched to as high as about 28 percent of oxygen by volume is introduced into the reactor from the bottom. Satisfactory dispersion and circulation of gas bubbles in the terephthalic acid oxidation slurry requires intense mechanical agitation due to the large volumetric flow rate of air or oxygen-enriched air stream. Since oxygen is in stoichiometric excess, the continuous gas phase formed in the reactor headspace contains residual oxygen.

The oxygen concentration in the headspace of the reactor must be maintained below the flammable limit, practically maintained below about eight to nine percent by volume for safe operation and typically less than about five percent by volume to prove a safe margin. The oxygen enrichment degree of the oxygen-enriched air based paraxylene oxidation process, practically as high as about 28 percent by volume, is limited by severe corrosion of the reactor material by oxygen in the present of Br in the liquid phase and limit of residual oxygen concentration in the continuous phase.

To eliminate the flammability of the gas vapor in the paraxylene oxidation reactor, prior methods such as those disclosed in US 5 371 283 produce purified terephthalic acid by pure oxygen in a liquid oxidation reactor in which oxygen is fed under the baffle and the interface between the gas phase and the liquid phase is eliminated. The design of this reactor though is more complex than conventional paraxylene oxidation reactors and further requires external nitrogen for dilution of the gas vapor in the reactor.

Alternatively, US 5 693 856 teaches oxygen enrichment and pure oxygen process for production of purified terephthalic acid with carbon dioxide recycle. This process will require additional devices for carbon dioxide separation and recycling.

In conventional oxygen-enriched air based purified terephthalic acid production processes, the volumetric flow rate of the air stream into the reactor is very high since air contains a large portion of inert nitrogen gas. The air stream is introduced into the liquid phase from the reactor bottom at a very high flow rate and the relatively large air bubbles are dispersed and circulated in the reactor by impellors.

Although the reactor design, such as the impellers and gas distributors can be optimized to enhance the oxygen efficiency, the reaction rate is still limited by the low oxygen partial pressure in the gas bubbles and the small specific interfacial surface area in the liquid phase. These cause high residual oxygen concentration in the continuous gas phase in the reactor headspace.

### Disclosure of the present invention: object, solution, advantages

Starting from the disadvantages and shortcomings as described above and taking the prior art as discussed into account, an object of the present invention is to overcome the problems that earlier methods have experienced.

This object is accomplished by a method comprising the features of claim 1; in particular, the present invention is able to overcome the inadequacies in the earlier terephthalic acid production methods by adding, in particular injecting, at least one additional or auxiliary oxygen flow into the reactor. Advantageous embodiments and expedient improvements of this improved terephthalic acid production method are disclosed in the dependent claims.

Adding, in particular injecting, at least one additional or auxiliary oxygen stream into the stirred paraxylene oxidation reactor where the production of terephthalic acid by the oxidation of paraxylene is occurring will improve the production of the terephthalic acid.

The additional or auxiliary stream of pure or near pure oxygen can advantageously be injected into the reactor through at least one gas diffuser and/or can expediently be injected as at least one additional or auxiliary oxygen stream and/or along with recycled solvent from the reactor.

In one embodiment of the present invention, there is disclosed an improved method for producing terephthalic acid comprising the steps of reacting paraxylene and oxygen in at least one air-based paraxylene oxidation stirred reactor in the presence of at least one catalyst, the improvement comprising adding, in particular injecting, at least one additional or auxiliary oxygen stream to the reactor.

The oxygen that is reacted with the paraxylene can favourably be fed directly to the reactor as at least one air stream. The reaction can preferably occur in the presence of at least one solvent, such as acetic acid, and/or in the presence of at least one catalyst, such as at least one Co-Mn-Br catalyst.

In the operation of the reaction, the solvent can advantageously be recycled.

In a further expedient embodiment of the present invention, the additional or auxiliary oxygen stream can be added to, in particular injected into, the reactor along with the, favourably recycled, solvent.

The additional or auxiliary oxygen stream when added to, in particular injected into, the reactor without the, favourably recycled, solvent can preferably be selected from the group consisting of pure oxygen, near pure oxygen, and oxygen-enriched air.

The additional or auxiliary oxygen stream may advantageously be preheated to a temperature roughly the same as the temperature of the air stream providing oxygen to the reactor.

The additional or auxiliary oxygen stream can expediently be added to, in particular injected into, the reactor at a volumetric flow rate of from about 0.1 percent to about twenty percent of the air flow rate of the stream providing oxygen to the reactor.

The additional or auxiliary oxygen stream can favourably be fed to the reactor through at least one gas diffuser and/or through at least one nozzle to disperse the additional or auxiliary oxygen stream into the terephthalic acid oxidation slurry that is formed in the reactor.

The separate feeding, in particular the separate addition or the separate injection, of the additional or auxiliary oxygen stream such as, for example, pure oxygen has many advantages:
First, the volumetric flow rate of the pure oxygen stream is small compared to the flow rate of the air stream. For the same degree of oxygen enrichment, such as about fifteen percent oxygen enrichment (about 24.2 percent oxygen in the air stream), the oxygen volumetric flow rate is only about 4.3 percent of the air flow rate which makes dispersion of the small oxygen bubbles in the liquid phase practical.
Second, the additional or auxiliary oxygen stream can be injected into the reactor with recycled solvent through at least one nozzle at an optimal position that inhibits excessive coagulation of small oxygen bubbles with large air bubbles and attachment of oxygen bubbles on the reactor wall. The oxygen efficiency can be significantly improved.
Third, operation of the oxygen enrichment paraxylene oxidation reactor is more flexible and safer because the response to the residual oxygen concentration in the gas vapor to the degree of oxygen enrichment is more direct.

Additionally or alternatively, the additional or auxiliary oxygen-containing gas can be instantly switched to nitrogen, to carbon dioxide and/or to at least one other inert gas, in particular to at least one venting gas released from the reactor, to keep the residual oxygen concentration within the safe operating range. This provides a proactive measure to maintain safe operation of the paraxylene reactor.

Another advantage of the inventive method is that most oxygen is directly dispersed into the downward flowing liquid which compensates for the upward liquid flow driven by the gas flowing upwardly in the stirred paraxylene oxidation reactor. Compared to the relatively low dissolved oxygen concentration in the downward liquid flow in an air-based paraxylene oxidation reactor, the small oxygen bubbles carried in this liquid flow can ensure fast oxidation of paraxylene and significantly decrease the paraxylene oxidation intermediate concentrations.

It is practical to reduce operating cost of the purified terephthalic acid manufacturer by lowering the temperature and/or the pressure in the air-based paraxylene oxidation reactor through feeding of additional or auxiliary oxygen because this method can provide additional reaction zones in the downward liquid flow and can reduce the intermediate concentrations, especially the 4-CBA (4-carboxybenzaldhyde) concentration.

Furthermore, the liquid phase combustion of acetic acid and the side reactions, which are more of a concern at high operating temperatures, can be reduced at a lower operating temperature without significant reduction of paraxylene use efficiency.

Additionally, the low impurities contained in terephthalic acid can reduce the loadings and operating costs of the subsequent processing procedures, such as treatment of the venting gas and hydrogen demand for purification further reducing costs.

Other benefits, such as the auxiliary mixing effect caused by gas injection for reduction of electricity consumption, are also provided by this new and inventive method. Therefore, this new and inventive method can provide for capacity improvement of current purified terephthalic acid production devices.

### Detailed description of the present invention;

### best way of embodying the present invention

In the present method to increase the amount of oxygen being fed to a reactor for producing terephthalic acid and improve the overall production of the terephthalic acid, an additional or auxiliary oxygen-containing gas stream is directly introduced into an air based paraxylene oxidation reactor aimed at increasing the terephthalic acid production capacity of the reactor.

After being advantageously preheated to a temperature close to the temperature of the air stream, the additional or auxiliary oxygen-containing gas stream is injected into the reactor through a set of gas diffusers and/or nozzles installed in the paraxylene oxidation reactor.

The gas flow rate of each individual gas diffuser and/or of each individual nozzle can be precisely controlled by a flow rate controller and promptly adjusted according the operating situation, such as the oxygen concentrations and/or temperatures in specific areas in the paraxylene oxidation reactor.

In order to prevent blockage of the gas distributor, at least one purging pipeline or at least one solvent pipeline can be installed on each gas diffuser and/or on each nozzle, through which acetic acid can be used to periodically purge the gas diffuser and/or the nozzle during operation. The purging pipeline(s) or the solvent pipeline(s) also can be used to prevent blockage of the gas diffuser and/or of the nozzle during startup and shutdown of the paraxylene oxidation reactor.

Alternatively, the oxygen-containing gas stream can be mixed with the recycled solvent recovered from at least one solvent condenser prior to being injected into the reactor. Compared to the flow rate of the air stream being injected into the paraxylene oxidation reactor, the volumetric flow rate of this stream is from about 0.1 percent to about twenty percent of the air flow rate.

The oxygen-containing gas stream is preferably injected into the circulating liquid stream of the paraxylene reactor through a set of gas diffusers and/or nozzles positioned in the reactor in a manner of accelerating dispersion of small oxygen gas bubbles in the liquid phase and reducing contact of the small bubbles with the reactor wall, such as beside or under at least one impeller of the reactor.

The additional or auxiliary oxygen-containing stream in either embodiment may be pure oxygen, nearly pure oxygen or oxygen-enriched air.

One advantage of the present invention is that the oxidation rate of paraxylene to terephthalic acid by oxygen is increased by separately introducing at least one pure or nearly pure oxygen stream in the paraxylene stirred oxidation reactor. This introduction can be through a set of gas diffusers and/or nozzles which are installed in an appropriate location within the paraxylene oxidation reactor.

The gas flow rate of each individual gas diffuser and/or of each individual nozzle can be precisely controlled by at least one flow rate controller. Compared to other paraxylene oxygen enrichment processes which mix the oxygen flow from the air flow prior to being injected into the reactor, oxygen can be dispersed into the terephthalic acid oxidation slurry as small gas bubbles by at least one high efficiency gas diffuser and/or by at least one nozzle because of its much lower volumetric flow rate compared to the air flow rate.

Due to the much higher partial pressure of oxygen in the small pure or near pure oxygen bubbles and the much higher specific surface area of the small oxygen gas bubbles in the terephthalic acid oxidation slurry, the oxygen efficiency of the pure or near pure oxygen flow is greatly improved.

In addition, since the oxygen is separately introduced into the paraxylene oxidation reactor, the residual oxygen concentration in the gas phase in the headspace of the reactor can be promptly controlled by adjusting the oxygen flow rate and/or by switching oxygen to nitrogen, to carbon dioxide or to at least one other inert gas to guarantee safe operation of the purified terephthalic acid oxidation process which has been a major concern in the paraxylene oxygen enrichment oxidation process.

Another problem associated with oxygen enrichment oxidation of paraxylene by the conventional Co-Mn-Br catalyst system is corrosion of the reactor, especially when the oxygen concentration in the air flow is high in the conventional oxygen enrichment oxidation process. This is a negative factor preventing high oxygen enrichment degree in conventional oxygen enrichment processes.

In the present invention, since the small amount of pure or near pure oxygen flow is separately introduced into the reactor and consumed quickly, an optimal arrangement of the at least one gas diffuser and/or of the at least one nozzle can avoid excess contact of the pure oxygen bubbles with the reactor wall and with other metal parts in the reactor. Thus, a high oxygen enrichment degree can be achieved to increase the output of the reactor while lessening the effects of corrosion.

While this invention has been described with respect to particular embodiments thereof, it is apparent that numerous other forms and modifications of the present invention will be obvious to those skilled in the art. The appended claims in this invention generally should be construed to cover all such obvious forms and modifications which are within the true spirit and scope of the present invention.

## Claims

1. A method for producing terephthalic acid, comprising the steps of:
- reacting paraxylene and oxygen in at least one air-based paraxylene oxidation stirred reactor in the presence of at least one catalyst, and
- adding, in particular injecting, at least one additional or auxiliary oxygen stream to the reactor.

2. The method according to claim 1 wherein the oxygen is directly fed to the reactor.

3. The method according to claim 1 or 2 wherein the catalyst is at least one Co-Mn-Br catalyst.

4. The method according to at least one of claims 1 to 3 wherein the additional or auxiliary oxygen stream is selected from the group consisting of pure oxygen, near pure oxygen, and oxygen-enriched air.

5. The method according to at least one of claims 1 to 4 wherein the additional or auxiliary oxygen stream is preheated to the temperature of the oxygen in the reactor.

6. The method according to at least one of claims 1 to 5 wherein the additional or auxiliary oxygen stream is fed to the reactor through at least one gas diffuser and/or through at least one nozzle.

7. The method according to at least one of claims 1 to 6 wherein the reaction occurs in the presence of at least one solvent.

8. The method according to claim 7 wherein said solvent is acetic acid.

9. The method according to claim 7 or 8 wherein the solvent is recycled from the reactor.

10. The method according to at least one of claims 7 to 9 wherein at least one solvent pipeline is installed on said gas diffuser and/or on said nozzle.

11. The method according to claim 10 wherein said solvent pipeline is for periodic acetic acid purging.

12. The method according to at least one of claims 1 to 11 wherein the flow of the additional or auxiliary oxygen stream is switched to at least one gas selected from the group consisting of nitrogen, carbon dioxide and inert gases.

13. The method according to claim 12 wherein said inert gases are venting gas released from the reactor.

14. The method according to at least one of claims 1 to 13 wherein the volumetric flow rate of the additional or auxiliary oxygen stream is from about 0.1 percent to about twenty percent of the air flow rate.

15. The method according to at least one of claims 1 to 14 wherein the additional or auxiliary oxygen stream is dispersed into at least one terephthalic acid oxidation slurry.
